Europäisches Patentamt

European Patent Office

Office européen des brevets

(11) Publication number: **0 056 001**
**B1**

## EUROPEAN PATENT SPECIFICATION

(45) Date of publication of patent specification: **11.12.85**

(21) Application number: **82200258.0**

(22) Date of filing: **21.02.79**

(60) Publication number of the earlier application in accordance with Art. 76 EPC: **0 003 906**

(51) Int. Cl.⁴: **B 01 J 10/02** // B01J16/00, B01J19/08, B01J35/00, C08F2/46, D01D5/18

(54) **Chemical process on the surface of a rotating body with subsequent discharge of the reaction product.**

(30) Priority: **21.02.78 GB 680678**

(43) Date of publication of application:
**14.07.82 Bulletin 82/28**

(45) Publication of the grant of the patent:
**11.12.85 Bulletin 85/50**

(84) Designated Contracting States:
**BE CH DE FR GB IT LU NL SE**

(56) References cited:
**CH-A- 595 133**
**FR-A- 990 163**
**GB-A-1 322 003**
**US-A-1 284 488**

(73) Proprietor: **IMPERIAL CHEMICAL INDUSTRIES PLC**
**Imperial Chemical House Millbank**
**London SW1P 3JF (GB)**

(72) Inventor: **Cowen, Geoffrey**
**132b Seneschal Square**
**Southgate Runcorn (GB)**
Inventor: **Norton-Berry, Philip**
**Tan-Y-Coed**
**Llandegla Clwyd (GB)**
Inventor: **Steel, Margaret Lilian**
**21 Bunbury Drive**
**Runcorn (GB)**

(74) Representative: **Hall, David Brian et al**
**Imperial Chemical Industries PLC Legal**
**Department: Patents Po Box 6**
**Welwyn Garden City Herts, AL7 1HD (GB)**

EP 0 056 001 B1

Courier Press, Leamington Spa, England.

## Description

This invention relates to chemical processes involving a centrifugal reactor.

It is known that in many chemical processes the reactants advantageously are spread as a thin film over a surface, and in this way reactions may be carried out with minimal heat transfer problems. One way of obtaining thin films of liquid reactants is to use a centrifugal reactor, which typically comprises a rapidly rotatable hollow body having an internal surface of rotation. The liquid reactant is supplied to the surface of the rotating body, where it is spread out over the surface by centrifugal forces generated by the rotation. An example of such a reactor is described in US 1284488, where it is used for the hydrogenation of unsaturated oils. The oils are fed to the surface of the rotating reactor, across which they are propelled as thin films of liquid in contact with gaseous hydrogen and catalytic or accelerating agents.

Conventional pumping or gravity feed systems can generally be used for feeding the liquids to such internal surfaces, but where the liquids are very viscous, this can present considerable difficulties. We have now found a way of spreading at least some of such liquids over a rotating surface of rotation thereby to provide them in a form particularly suitable for chemical treatment.

According to the present invention there is provided a process for carrying out a chemical reaction on a liquid capable of exhibiting the Weissenberg effect, comprising rotating a body having a surface of rotation coaxial with the axis of rotation, the surface of rotation extending from a supply zone to a discharge zone, being an external surface at least in the region of its supply zone and having its discharge zone at a position of maximum radius, dipping the rotating supply zone into the liquid thereby to cause the liquid to flow up over the surface by the Weissenberg effect until it reaches the discharge zone there to be flung from the position of maxium radius by centrifugal forces, and effecting the chemical reaction as the liquid flows over the rotating surface between the supply zone and the discharge zone.

The Weissenberg effect is that by which certain, generally viscous, liquids will climb up the surface of rotating bodies dipped into them, and spread upwards over their surfaces. The height climbed will depend both on the physical properties of the liquid and on the dimensions and rate of rotation of the body. It is an effect which can often (annoyingly) occur when stirring certain very viscous liquids at high speed, and is referred to, for example, in The Flow of High Polymers by Stanley Middleman, Interscience Publishers, 1968, page 41, and in J. Applied Polymer Science, Vol 8, pp 2339—2357 (1961). Where the radius becomes a maximum, further flow of the liquid would require it to travel inwards, against the centrifugal forces applied to it. A suitably sharp lip at the discharge zone can then cause the climbing liquid to separate and become flung off the surface under the centrifugal forces.

A preferred method is one in which the surface of rotation lies parallel to the axis in the region of the supply zone but becomes substantially radial before reaching the discharge zone.

A method which is particularly useful when reacting the Weissenberg-exhibiting liquid with a liquid or slurry that does not exhibit the effect, is one wherein the surface of rotation on extending from the supply zone to the discharge zone is initially an external surface but becomes folded back on itself to provide an internal surface of greater radius than the external surface before reaching the discharge zone, the liquid being reacted at least while flowing over the internal surface towards the discharge zone.

The product may be discharged from the surface either as a continuous sheet (which may be cooling or evaporating change from the liquid to the solid state) as fibres or filaments or as an atomised spray which if desired may be collected in bulk form as either a liquid or powder. It is convenient to place collecting apparatus around the rotating body to receive the product, the apparatus taking whatever form is appropriate to the type of product obtained. The process of the invention is particularly suitable for the production of either insoluble polymeric products in particulate form or in fibre form for example either long filaments or staple fibre especially a tangled wool, tow or non-weave fabric of fibres may readily be obtained from the discharge zone in many polymeric reactions.

If a body is rotating and liquid reactants are fed continuously to the surface of the body the movement of the liquid across the surface to the discharge zone and the discharge of products (and any excess reactants) will continue while the body is rotating. It may be necessary to apply trial and error judgements in order to arrange the balance of parameters for optimum yield of product but it may be seen that a continuous chemical production process may be conducted with the simplest of apparatus. The process has many advantages. The reaction takes place in a thin film and therefore contact with gas in the surrounding atmosphere or contact with solids on the surface is rapid and complete. Heat transfer problems are minimised because there is immediate and virtually complete contact by all portions of the reactant medium with a heat transfer surface, either the gaseous interface with the outer surface of the film or the contact of the underside of the film with the rotating body itself. Heat may be imparted or taken out of the film quikly and easily by either route and additionally if desired the film could be heated in a variety of ways e.g. infra-red and microwave radiation and dielectric or eddy current heating.

A further advantage of the process is that unstable or intermediate products and products capable of further reaction may be isolated rapidly and prevented from further reaction or decomposition by their rapid discharge from the surface by centrifugal force. They are thereby removed from the reaction zone and thus also can be removed from the influence of reaction conditions which might cause the products themselves to decompose. Solvent and any volatile byproducts are removed quickly by evaporation in the atmosphere after discharge.

The surface may be ridged, undulating, convoluted or otherwise patterned if desired in order to improve the movement of the reactant medium and/or the product for example to aid the mixing of components. The edge or rim of the body may be fluted or otherwise patterned in order to improve the discharge of product in any desired form.

The surface of rotation on which the chemical process of this invention takes place may be usefully an inert surface for example of glass, plastic, ceramic, metal, or composite materials especially chemically resistant metals e.g. stainless steel, nickel, titanium and tantalum are preferred. Alternatively metals coated with chemically resistant surface materials for example glass, silicone resins or polytetrafluoroethylene are usually employed. Flexible films may be used as materials for the rotating body which would be rigidified by the centrifugal force operating when they are rotating and they may constitute a cheap form of bowl or cylinder on which the process of this invention may be conducted.

With the materials mentioned above, the surface of the rotating body takes no part in the chemical reaction but it may be advantageous to conduct the process of the invention on a surface which reacts chemically with the reactant medium. Most usefully the surface may be treated with a catalyst for the chemical reaction in question and in this way the residence time necessary to obtain complete reaction and a high yield of product may be reduced and the throughput of the process thereby increased. For example the surface of the reactor may be coated with an adherent porous material (for example high area silica or alumina) and the surface coating may itself act as a catalyst or constitute a substrate on which catalystic materials may easily be deposited or adsorbed.

Some chemical reactions are initiated or conducted photochemically e.g. by UV light but hitherto photochemical processes have not been industrially successful because of the difficulty in obtaining a high light intensity uniformly throughout a large mass or over a large area of surface reactant. In the process of the present invention this problem is minimised because although the reaction is spread over a surface area the surface is rotating at high speed and therefore a high intensity beam of light e.g. from a laser may be used and focussed upon a small region of that surface. The fact that the surface is rotating at high speed and carries the reactant medium also on a rotary path allows a large area to be illuminated almost instantaneously by the light beam. In addition the movement of the reactant medium in an axial direction or with an axial component to the direction ensures the illumination of an even greater mass of reactant by the light beam.

The pencil beam of a laser source may usefully be focussed onto a portion of the discharge zone (for example a rim or flange around a radial surface) and may be used to modify the product as it is discharged. For example a polymeric product may be cross-linked thereby and thus stabilised in a desired form, especially a fibrous or particulate form, very conveniently and uniformly without the need for a subsequent treatment. Altenatively the source may emit high energy radiation, for example shortwave electromagnetic such as X-rays or γ-rays, or high energy particles such as electrons α-particles or ion beams, may be used if desired to conduct the process.

To illustrate the invention, specific embodiments of the two preferred shapes referred to above are shown in the accompanying drawings, in which

Figure 1 is a section through a rotating body having an external surface which becomes a radial surface in the region of the discharge zone, and

Figure 2 is a section through a further rotating body in which the surface folds back on itself to become an internal surface.

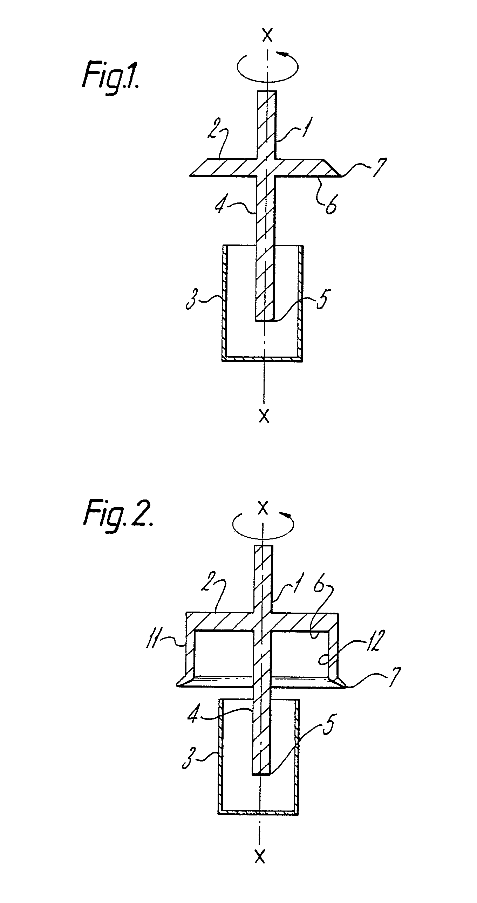

In Figure 1 the rotating body comprises a solid spindle 1 having a disc 2 mounted part way along its length. The body is rotatable about an axis XX, and the lower end dips into a container 3 for holding the liquid. The spindle has an external surface of rotation 4 parallel to the axis of rotation. The lower end 5 which dips into the container, provides a supply zone. The under surface of the disc 6 is a radial surface, and at the perimeter of the disc, the surface diameter is at a maximum, providing the discharge zone of the surface.

In use the liquid is placed in the container. The body is rotated at high speed and the supply zone dipped into the liquid. This climbs up the spindle according to the Weissenberg effect until it reaches the radial undersurface of the disc. There is flows outwards to be flung from the periphery by centrifugal forces. The reaction is preferably carried out as the liquid climbs up the external surface, this being the part whereon it is most readily controlled. However, where thinner films of the liquid are preferred or a rapid passage through a heated zone is desired for an unstable liquid for example, the reaction can be carried out on the radial surface as the liquid spreads out in its journey to the discharge zone. Under both reaction conditions the viscous liquid is pumped up out of the container by the rotation of the dipped end of the spindle.

In Figure 2 many parts are essentially the same and like parts have been given like numerals. Thus a spindle 1 rotatable about the axis XX, has a disc 2 providing a radial surface. However around the outside of the disc is a cylinder 11 providing an internal surface 12, ending in a discharge zone 7 at its lower end. As in the previous embodiment, the supply zone at the lower end of the spindle dips into the liquid, and as it spins it pumps the liquid up its external surface 4. On reaching the radial surface 6 of the disc it spreads outwards under centrifugal forces until it strikes the internal surface 12. Centrifugal forces press the liquid against the internal surface and cause it to spread out. As further liquid is pumped up the spindle to the internal surface it forces the earlier liquid toward the discharge zone, for discharge as before.

This configuration is useful in providing a surface for supporting Newtonion liquids, enabling them to be reacted with the viscous non-Newtonion liquid pumped up from the container. However, other reactions can also be carried out on the external surface 4 of the spindle or on the radial surface 6 of the disc in essentially the same manner as those carried out in the apparatus shown in Figure 1.

**Claims**

1. A process for carrying out a chemical reaction on a liquid medium comprising rotating a body having a surface of rotation coaxial with the axis of rotation, the surface of rotation extending from a supply zone to a discharge zone, dipping the rotating supply zone into the liquid medium thereby to cause at least a portion thereof to flow up over the surface towards the discharge zone, effecting the chemical reaction as the liquid medium flows over the rotating surface between the supply zone and the discharge zone, causing the liquid medium to be flung from the discharge zone by centrifugal forces, characterised in that the body has only an external surface of rotation at least in the region of the supply zone and in that the liquid medium is one capable of exhibiting the Weissenberg effect, whereby when the supply zone of the rotating body is dipped into the liquid medium the portion thereof is caused to flow upwards over the external surface of the body and be reacted as it travels towards the discharge zone.

2. A process as claimed in claim 1 in which the surface of rotation lies parallel to the axis in the region of the supply zone but becomes substantially radial before reaching the discharge zone.

3. A process as claimed in claim 2 wherein the surface of rotation on extending from the supply zone to the discharge zone is initially an external surface but becomes folded back on itself to provide an internal surface of greater radius than the external surface before reaching the discharge zone, the portion of liquid being reacted at least while flowing over the internal surface towards the discharge zone.

**Revendications**

1. Procédé pour conduire une réaction chimique sur un milieu liquide consistant à faire tourner un corps qui présente une surface de rotation dont l'axe coïncide avec l'axe de rotation, la surface de rotation s'étendant dans une zone d'alimentation à zone de décharge, à plonger la zone d'alimentation en rotation dans le milieu liquide afin qu'au moins une partie de celui-ci s'élève sur la surface vers la zone de décharge, à effectuer la réaction chimique pendant que le milieu liquide s'écoule sur la surface en rotation entre la zone d'alimentation et la zone de décharge, à provoquer une projection du produit de réaction à partir de la zone de décharge sous l'effet de forces centrifuges et à recueillir le produit de réaction ainsi projeté, caractérisé en ce que le corps présente uniquement une surface extérieure de rotation au moins dans la région de la zone d'alimentation et en ce que le milieu liquide est un milieu capable de présenter l'effet Weissenberg, de manière que, lorsque la zone d'alimentation du corps en rotation est plongée dans le milieu liquide, une partie de ce dernier s'élève sur la surface extérieure du corps et entre en réaction en progressant vers la zone de décharge.

2. Procédé selon la revendication 1, dans lequel la surface de rotation s'étend parallèlement à l'axe dans la région de la zone d'alimentation, mais devient sensiblement radiale avant d'atteindre la zone de décharge.

3. Procédé selon la revendication 2, dans lequel la surface de rotation, dans son étendue de la zone d'alimentation à la zone de décharge, est initialement une surface extérieure mais devient repliée sur elle-même pour former une surface intérieure de plus grand rayon que la surface extérieure avant d'atteindre la zone de décharge, la partie du liquide réagissant au moins pendant qu'elle s'écoule sur la surface intérieure de la zone de décharge.

**Patentansprüche**

1. Verfahren zur Durchführung einer chemischen Reaktion an einem flüssigen Medium, bei dem
ein Körper mit einer Rotationsfläche, die mit der Rotationsachse koaxial ist, rotieren gelassen wird, wobei sich die Rotationsfläche von einer Zuführungszone zu einer Ablaßzone erstreckt,
die rotierende Zuführungszone in das flüssige Medium eingetaucht wird, um dadurch zu veranlassen, daß mindestens ein Anteil des flüssigen Mediums über die Rotationsfläche nach oben in Richtung auf die Ablaßzone fließt,
die chemische Reaktion bewirkt wird, während das flüssige Medium zwischen der Zuführungszone und der Ablaßzone über die Rotationsfläche fließt,
durch Zentrifugalkräfte veranlaßt wird, daß das Reaktionsprodukt von der Ablaßzone weggeschleudert wird, und
das so weggeschleuderte Reaktionsprodukt gesammelt wird,
dadurch gekennzeichnet, daß der Körper mindestens in dem Bereich der Zuführungszone nur eine Außen-Rotationsfläche aufweist und daß das flüssige Medium dazu befähigt, ist, den Weissenberg-Effekt zu zeigen, wodurch, wenn die Zuführungszone des rotierenden Körpers in das flüssige Medium eingetaucht ist, der Anteil des flüssigen Mediums dazu veranlaßt wird, über die Außenfläche des Körpers nach oben zu fließen und zur Reaktion gebracht zu werden, während er sich in Richtung auf die Ablaßzone bewegt.

2. Verfahren nach Anspruch 1, bei dem die Rotationsfläche in dem Bereich der Zuführungszone parallel zu der Rotationsachse liegt, jedoch vor dem Erreichen der Ablaßzone einen im wesentlichen radialen Verlauf annimmt.

3. Verfahren nach Anspruch 2, bei dem die Rotationsfläche in ihrem Verlauf von der Zuführungszone zu der Ablaßzone anfänglich eine Außenfläche ist, jedoch vor dem Erreichen der Ablaßzone umbiegt, um eine Innenfläche bereitzustellen, die einen größeren Radius hat als die Außenfläche, wobei der Anteil des flüssigen Mediums mindestens zur Reaktion gebracht wird, während er über die Innenfläche in Richtung auf die Ablaßzone fließt.

*Fig.1.*

*Fig.2.*

1